# EUROPEAN PATENT SPECIFICATION

(11) **EP 3 912 668 B1**
(45) Date of publication and mention of the grant of the patent: **04.10.2023**
(21) Application number: 20741470.7
(22) Date of filing: 16.01.2020
(51) Int. Cl.: A61M 25/08, A61M 25/09

(54) **ELONGATED MEDICAL INSTRUMENT DRIVING DEVICE AND MEDICAL DEVICE**
ANSTEUERVORRICHTUNG FÜR EIN LÄNGLICHES MEDIZINISCHES INSTRUMENT UND MEDIZINPRODUKT
DISPOSITIF D'ENTRAÎNEMENT D'INSTRUMENT MÉDICAL ALLONGÉ ET DISPOSITIF MÉDICAL

(30) Priority: 17.01.2019 US 201962793640 P
(43) Date of publication of application: 24.11.2021
(73) Proprietor: ASAHI INTECC CO., LTD., Seto-shi, Aichi 489-0071 (JP)
(72) Inventor: FURUKAWA Muneya, Tustin, California 92780 (US)
(74) Representative: Prinz & Partner mbB
(86) International application number: PCT/JP2020/001315
(87) International publication number: WO 2020/149369

(56) References cited:
- JP-A- H08 512 225
- JP-A- 2014 200 287
- US-A1- 2010 234 873
- US-A1- 2011 184 390
- US-A1- 2012 203 168
- US-A1- 2015 297 864
- US-A1- 2018 311 473
- US-B2- 8 764 766

## Description

### TECHNICAL FIELD

The technology disclosed herein relates to a driving device for an elongated medical instrument as represented by a medical guide wire or catheter; and a medical device including the driving device.

### BACKGROUND ART

When a narrow segment (an affected area) occurs in a body lumen such as a blood vessel, various therapeutic medical instruments as represented by, for example, a balloon catheter may be used. For guiding such a therapeutic medical instrument to a narrow segment, a medical practitioner delivers a medical guide wire (an elongated medical instrument) to the narrow segment.

By the way, in a case that the affected area became a totally occluded (which may also be referred as "CTO affected area ") due to a calcification, it may be difficult to insert the guide wire into the CTO affected area. If that is the case, a medical practitioner may hold with her/his hand the rear-end side (i.e. the base end side of an elongated medical instrument, which may also be referred to as the hand side) of an elongated medical instrument as represented by a medical guide wire or catheter (which may also be referred to as a penetrating catheter), and then apply a relatively large force to push the front-end portion thereof into CTO lesion (Patent Literature 1, for example).

In other cases, for example, a guide wire may be made to intrude into a false lumen (between the inner membrane and outer membrane of a blood vessel) so as to avoid a narrow segment in the blood vessel, and after passing the narrow segment, the front end of the guide wire is then made to penetrate the inner membrane of the blood vessel to re-enter (which may also be referred to as reentry) a true lumen (the inner cavity of a blood vessel). When causing the front end of the guide wire penetrate the inner membrane of the blood vessel for reentry into the true lumen, a medical practitioner may hold with her/his hand the rear-end side of the guide wire, and then apply a relatively large force to push the front end thereof into the true lumen.

### CITATION LIST

### PATENT LITERATURE

Patent Literature 1: JP Patent Application Publication No. 2018-79247
US 2010/234873 A1 discloses a device for driving an elongate member, the driving device having a clamping mechanism with which the pinching force applied by driving wheels onto the elongate member can be adjusted.
Other documents are US 2011/184390 A1, US 2018/311473 A1, US 8 764 766 B2, US 2012/103168 A1 and US 2015/297864 A1.

### SUMMARY OF INVENTION

### TECHNICAL PROBLEM

It has not been proposed a device which is capable of easily applying a large force to an elongated medical instrument and of which configuration is simple. Such the device has been demanded.

An object of the invention is to provide a device which is capable of easily applying a large force to an elongated medical instrument and of which configuration is simple.

### SOLUTION TO PROBLEM

The technology disclosed herein provides an elongated medical instrument-driving device capable of applying a predetermined force or a predetermined velocity to an elongated medical instrument as defined in claim 1; and a medical device including the device as defined in claim 9.

It is noted that the term "front-end side (forward)" as used in the descriptions presented below means a direction from the rear end toward the front end along the axis direction (longitudinal direction) of an elongated medical instrument. Similarly, the term "rear-end side (backward)" means a direction opposite to the front-end side, i.e., a direction from the front end toward the rear end along the axis direction (longitudinal direction) of the elongated medical instrument.

In other words, the term "front-end side (forward)" means a direction from the side of a drive mechanism included in an elongated medical instrument-driving device described below toward the side of a supporting member included in the elongated medical instrument-driving device along the axis direction (longitudinal direction) of the supporting member. Similarly, the term "rear-end side (backward)" means a direction opposite to the front-end side, i.e., a direction from the supporting member included in the elongated medical instrument-driving device toward the side of the drive mechanism along the axis direction (longitudinal direction) of the supporting member.

The technology disclosed herein is summarized as follows.

### Embodiment A-1: Elongated medical instrument-driving device

An elongated medical instrument-driving device, including: a supporting member for supporting an elongated medical instrument having a front end and a rear end; and a drive mechanism for applying a driving force to the supporting member so that the elongated medical instrument moves toward a front-end side, the drive mechanism having an elastic body.

### Embodiment A-2: Elongated medical instrument-possessing medical device

An elongated medical instrument-possessing medical device, including: an elongated medical instrument having a front end and a rear end; a supporting member for supporting the elongated medical instrument; and a drive mechanism for applying a driving force to the supporting member so that the elongated medical instrument moves toward a front-end side, the drive mechanism including an elastic body.

### Example A-3:

The device can be used in a non-claimed method including the steps of: providing an elongated medical instrument-possessing medical device including an elongated medical instrument having a front end and a rear end, a supporting member for supporting the elongated medical instrument, and a drive mechanism for applying a driving force to the supporting member so that the elongated medical instrument moves toward a front-end side, the drive mechanism including an elastic body; supporting the elongated medical instrument with the supporting member; and forcing the elongated medical instrument to move toward the front-end side by compressing or stretching the elastic body of the drive mechanism, and then applying a restoring force of the compressed or stretched elastic body to the supporting member.

### Embodiments A-1, A-2, and example

A-3 each may further have one or two or more of the following configurations (a1), (a2), and (a3).
(a1) The drive mechanism is for applying an impact force or an impulse force to the supporting member.
(a2) The drive mechanism is for applying a driving force to the supporting member so that the velocity of the supporting member falls within a range between 1 m/s and 30 m/s.
(a3) The drive mechanism is for applying a driving force to the supporting member, the driving force being large enough to enable the supporting member to move further toward the front-end side when application of the driving force to the supporting member is stopped.

### Embodiment B-1: Elongated medical instrument-driving device

An elongated medical instrument-driving device, including: a supporting member for supporting an elongated medical instrument having a front end and a rear end; and a drive mechanism for applying a driving force to the supporting member so that the elongated medical instrument moves toward a front-end side, the drive mechanism being for applying an impact force or an impulse force to the supporting member.

### Embodiment B-2: Elongated medical instrument-possessing medical device

An elongated medical instrument-possessing medical device, including: an elongated medical instrument having a front end and a rear end; a supporting member for supporting the elongated medical instrument; and a drive mechanism for applying a driving force to the supporting member so that the elongated medical instrument moves toward a front-end side, the drive mechanism being for applying an impact force or an impulse force to the supporting member.

### Example B-3:

The device can be used in a non-claimed method including the steps of: providing an elongated medical instrument-possessing medical device including an elongated medical instrument having a front end and a rear end, a supporting member for supporting the elongated medical instrument, and a drive mechanism for applying a driving force to the supporting member so that the elongated medical instrument moves toward a front-end side, the drive mechanism being for applying an impact force or an impulse force to the supporting member; supporting the elongated medical instrument with the supporting member; and forcing the elongated medical instrument to move toward the front-end side by applying the impact force or the impulse force from the drive mechanism.

Embodiments B-1, B-2, and example B-3 each may further have one or two or more of the following configurations (b1), (b2), and (b3).
(b1) The drive mechanism includes an elastic body.
(b2) The drive mechanism is for applying a driving force to the supporting member so that the velocity of the supporting member falls within a range between 1 m/s and 30 m/s.
(b3) The drive mechanism is for applying a driving force to the supporting member, the driving force being large enough to enable the supporting member to move further toward the front-end side when application of the driving force to the supporting member is stopped.

### Embodiment C-1: Elongated medical instrument-driving device

An elongated medical instrument-driving device, including: a supporting member for supporting an elongated medical instrument having a front end and a rear end; and a drive mechanism for applying a driving force to the supporting member so that the elongated medical instrument moves toward a front-end side, the drive mechanism being for applying a driving force to the supporting member so that the velocity of the supporting member falls within a range between 1 m/s and 30 m/s.

### Embodiment C-2: Elongated medical instrument-possessing medical device

An elongated medical instrument-possessing medical device, including: an elongated medical instrument having a front end and a rear end; a supporting member for supporting the elongated medical instrument; and a drive mechanism for applying a driving force to the supporting member so that the elongated medical instrument moves toward a front-end side, the drive mechanism being for applying a driving force to the supporting member so that the velocity of the supporting member falls within a range between 1 m/s and 30 m/s.

### Example C-3:

The device can be used in a non-claimed method including the steps of: providing an elongated medical instrument-possessing medical device including an elongated medical instrument having a front end and a rear end, a supporting member for supporting the elongated medical instrument, and a drive mechanism for applying a driving force to the supporting member so that the elongated medical instrument to move toward a front-end side, the drive mechanism being for applying a driving force to the supporting member so that the velocity of the supporting member falls within a range between 1 m/s and 30 m/s; supporting the elongated medical instrument with the supporting member; and forcing the elongated medical instrument to move toward the front-end side by applying the driving force of the drive mechanism to the supporting member.

Embodiments C-1, C-2, and example C-3 each may further have one or two or more of the following configurations (c1), (c2), and (c3).
(c1) The drive mechanism includes an elastic body.
(c2) The drive mechanism is for applying an impact force or an impulse force to the supporting member.
(c3) The drive mechanism is for applying a driving force to the supporting member, the driving force being large enough to enable the supporting member to move further toward the front-end side when application of the driving force to the supporting member is stopped.

### Embodiment D-1: Elongated medical instrument-driving device

An elongated medical instrument-driving device, including: a supporting member for supporting an elongated medical instrument having a front end and a rear end; and a drive mechanism for applying a driving force to the supporting member so that the elongated medical instrument moves toward a front-end side, the drive mechanism being for applying a driving force to the supporting member, the driving force being large enough to enable the supporting member to move further toward the front-end side when application of the driving force to the supporting member is stopped.

### Embodiment D-2: Elongated medical instrument-possessing medical device

An elongated medical instrument-possessing medical device, including: an elongated medical instrument having a front end and a rear end; a supporting member for supporting the elongated medical instrument; and a drive mechanism for applying a driving force to the supporting member so that the elongated medical instrument moves toward a front-end side, the drive mechanism being for applying a driving force to the supporting member, the driving force being large enough to enable the supporting member to move further toward the front-end side when application of the driving force to the supporting member is stopped.

### Example D-3:

The device can be used in a non-claimed method including the steps of: providing an elongated medical instrument-possessing medical device including an elongated medical instrument having a front end and a rear end, a supporting member for supporting the elongated medical instrument, and a drive mechanism for applying a driving force to the supporting member so that the elongated medical instrument moves toward a front-end side, the drive mechanism being for applying a driving force to the supporting member, the driving force being large enough to enable the supporting member to move further toward the front-end side when application of the driving force to the supporting member is stopped; supporting the elongated medical instrument with the supporting member; and forcing the elongated medical instrument to move toward the front-end side by applying the driving force of the drive mechanism to the supporting member.

Embodiments D-1, D-2, and example D-3 each may further have one or two or more of the following configurations (d1), (d2), and (d3).
(d1) The drive mechanism includes an elastic body.
(d2) The drive mechanism is for applying an impact force or an impulse force to the supporting member.
(d3) The drive mechanism is for applying a driving force to the supporting member so that the velocity of the supporting member falls within a range between 1 m/s and 30 m/s.

Embodiments A-1, A-2, B-1, B-2, C-1, C-2, D-1, D-2, and examples A-3, B-3, C-3 and D-3 each may further have one or two or more of the following configurations (x1), (x2), (x3), and (x4).
(x1) A travel distance d of the supporting member is set at a value satisfying the expression: 0 mm < d < 15 mm.
(x2) The supporting member is separable or detachable from the drive mechanism (has a configuration separable or detachable from the drive mechanism).
(x3) The elongated medical instrument is a medical guide wire or catheter.
(x4) The elastic body is a rubber string, a coil spring, or a flat spring.

### BRIEF DESCRIPTION OF DRAWINGS

Fig. 1 shows a top view of the configuration of an elongated medical instrument-driving device (Embodiment A-1).
Fig. 2 shows a side view of the elongated medical instrument-driving device as shown in Fig. 1.
Fig. 3 shows a top view of the elongated medical instrument-driving device as 10 shown in Fig. 1 where an elastic body of a drive mechanism is stretched.
Fig. 4 shows a top view of the elongated medical instrument-driving device as shown in Fig. 3 where an elongated medical instrument is moved toward the front-end side by applying a restoring force of the stretched elastic body of the drive mechanism to a supporting member.
Fig. 5 shows a top view for illustrating an alternative method of using the elongated medical instrument-driving device and an elongated medical instrument medical device as shown in Fig. 1.
Fig. 6 shows a top view of a modified example (Modified Example A1) of the elongated medical instrument-driving device and the elongated medical instrument-possessing medical device as shown in Fig. 1.
Fig. 7 shows a top view of another modified example (Modified Example A2) of the elongated medical instrument-driving device and the elongated medical instrument-possessing medical device as shown in Fig. 1.
Fig. 8 shows a top view of yet another modified example (Modified Example A3) of the elongated medical instrument-driving device and the elongated medical instrument-possessing medical device 100 as shown in Fig. 1.
Fig. 9 shows a side view of an elongated medical instrument-driving device as shown in Fig. 8.
Fig. 10 shows a top view of the configuration of an elongated medical instrument-driving device (Embodiment B-1).
Fig. 11 shows a top view of the configuration of an elongated medical instrument-driving device (Embodiment C-1).
Fig. 12 shows a top view of the configuration of an elongated medical instrument-driving device (Embodiment D-1).

### DESCRIPTION OF EMBODIMENTS

### Embodiment A-1: Elongated medical instrument-driving device

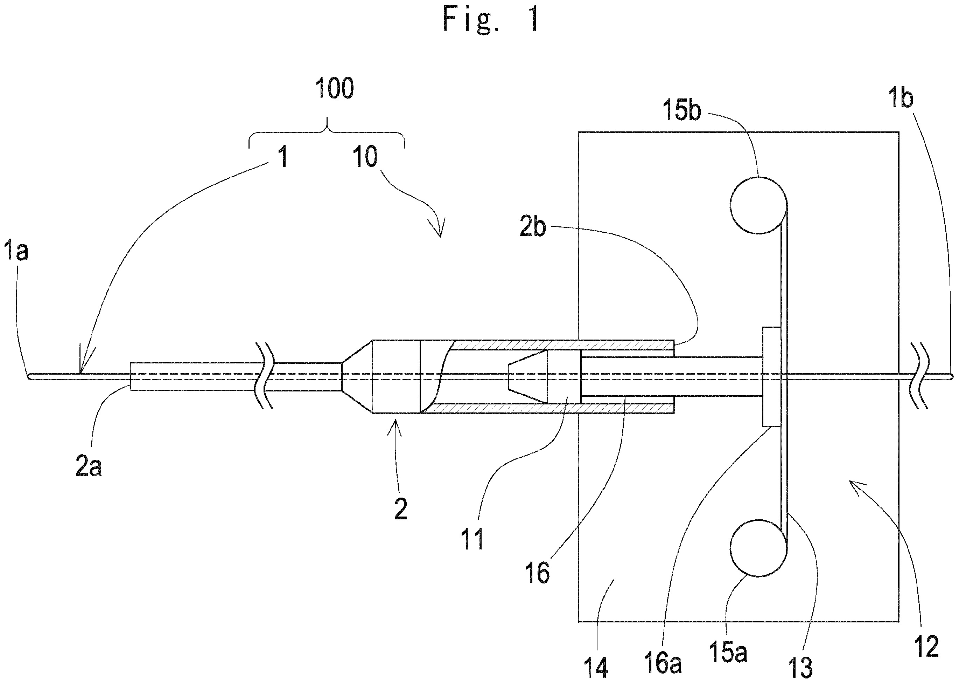

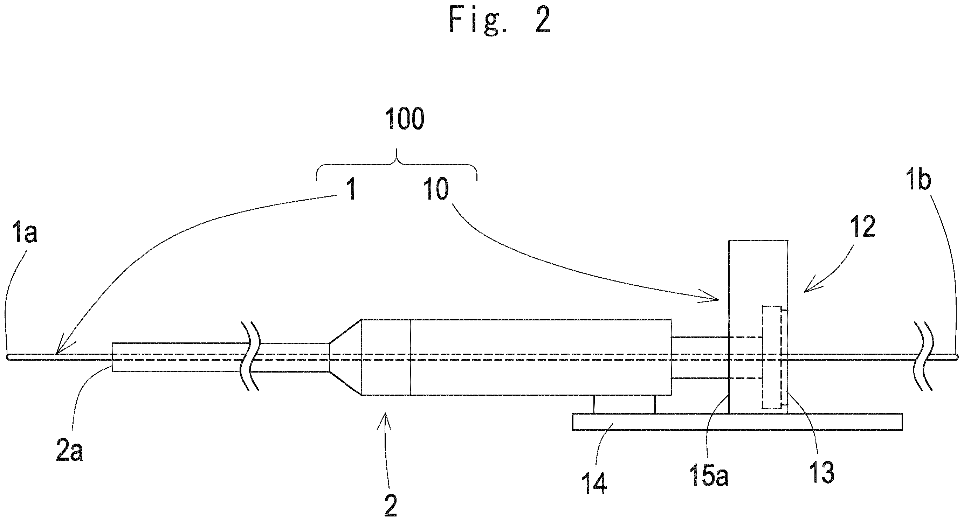

Fig. 1 shows a top view of the configuration of an elongated medical instrument-driving device. Fig. 2 shows a side view of the elongated medical instrument-driving device 10 of Fig. 1. The elongated medical instrument-driving device 10 of Fig. 1 includes a supporting member 11 for supporting (or holding) an elongated medical instrument 1, and a drive mechanism 12 for applying a driving force to the supporting member 11 so that the elongated medical instrument 1 moves toward a front-end side. The elongated medical instrument 1 has a front end 1a and a rear end 1b.

The drive mechanism 12 has an elastic body 13 (includes the elastic body 13).

A medial guide wire is used as the elongated medical instrument 1. Detailed description of the medical guide wire, which is well known, shall be omitted. A known elongated medical instrument such as a medical guide wire or a medical catheter may be used for the elongated medical instrument.

The elongated medical instrument-driving device 10 is seated, for example, on a surface of a supporting plate 14. A pair of supporting pillars 15a and 15b are disposed on the surface of the supporting plate 14. There is no particular limitation for materials of the supporting plate 14 and the pair of supporting pillars 15a and 15b, but resin materials and metal materials may be used.

The elastic body 13 is supported between the pair of supporting pillars. A rubber string is used as the elastic body 13. One end of the elastic body 13 is fixed to the supporting pillar 15a, and the other end is fixed to the supporting pillar 15b.

A known elastic body such as a rubber string, a flat spring, and a coil spring may be used as the elastic body. There is no particular limitation for the material of the elastic body, but a resin material and a metal material may be used.

There is no particular limitation for the configuration of the supporting member 11 as long as it can support (or hold) the elongated medical instrument 1. A support (commonly called a torquer) to be attached to an elongated medical instrument, with which a medical practitioner can operate the elongated medical instrument 1, may be used as the supporting member 11. Instead of the above support called a torquer, a support having a configuration similar to that of a chuck for holding a tool such as a drill may be used as the supporting member.

The supporting member may be permanently fixed to the elongated medical instrument or may be temporarily fixed so as to be able to be removed as required.

The supporting member may have a ring-like or flange-like shape which can be disposed around the elongated medical instrument. Examples of the ring-like (or flange-like) shape include a shape having a slit formed at a portion in the circumferential direction, such as a C-like shape.

The supporting member 11 includes a rod 16 at the rear-end side thereof. The rod 16 has a flange 16a at the rear-end side thereof.

There is no particular limitation for materials of the supporting member 11 and the rod 16, but resin materials and metal materials may be used.

The elongated medical instrument-driving device 10 is used in a state where the elongated medical instrument 1 is supported by the supporting member 11. As shown in Fig. 1, the elongated medical instrument-driving device 10 may be used in a state where it is received in the inside of a medical catheter 2 so that the elongated medical instrument 1 can move easily.

The medical catheter 2 has a front end 2a and a rear end 2b. Detailed description of the configuration of the medical catheter, which is well known, shall be omitted. A known catheter, such as a guiding catheter and a micro catheter, may be used as the medical catheter.

### Embodiment A-2: Elongated medical instrument-possessing medical device

An elongated medical instrument-possessing medical device 100 includes the elongated medical instrument 1 and the elongated medical instrument-driving device 10 shown in Fig. 1.

The elongated medical instrument-possessing medical device 100 includes the elongated medical instrument 1 having the front end 1a and the rear end 1b, the supporting member 11 for supporting (or holding) the elongated medical instrument 1, and the drive mechanism 12 for applying a driving force to the supporting member 11 so that the elongated medical instrument 1 moves toward the front-end side, the drive mechanism 12 including the elastic body 13.

The configurations of the elongated medical instrument 1 and the elongated medical instrument-driving device 10 are as described above.

### Example A-3: Method of driving elongated medical instrument (not being part of the invention)

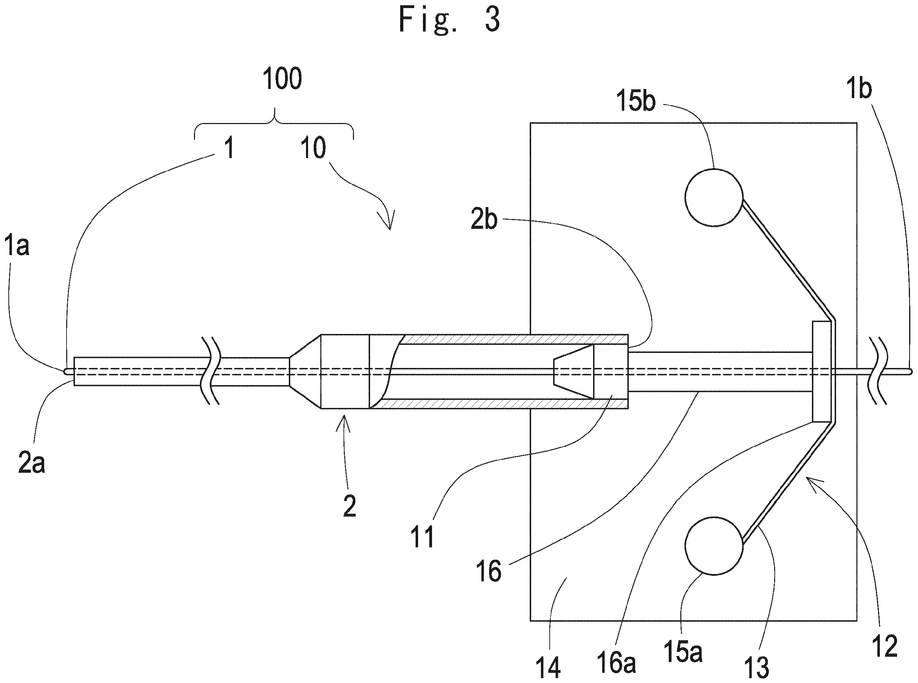

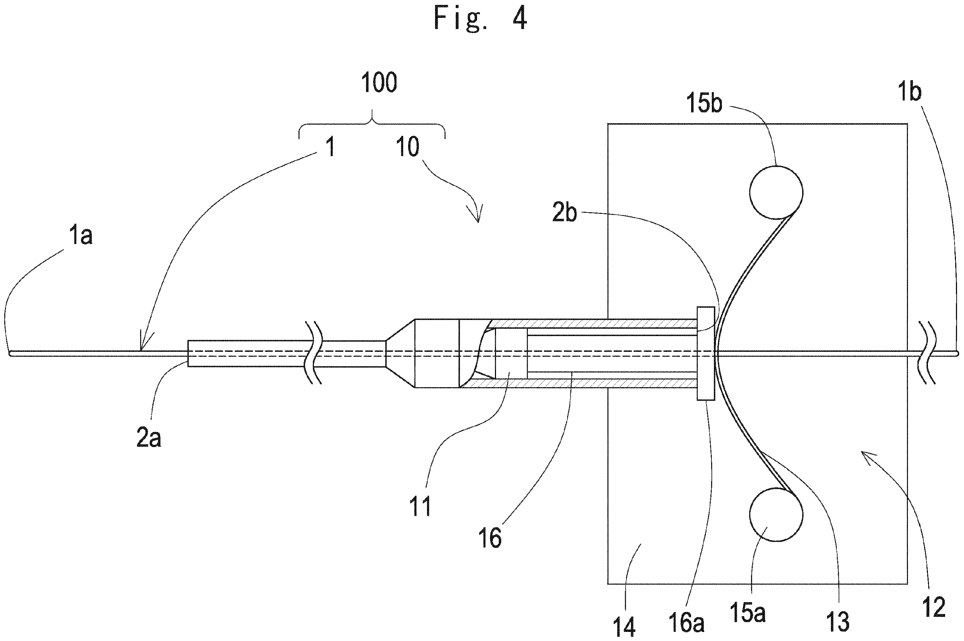

A method of driving an elongated medical instrument includes the steps of: providing the elongated medical instrument-possessing medical device 100 including the elongated medical instrument 1 having the front end 1a and the rear end 1b, the supporting member 11 for supporting (or holding) the elongated medical instrument 1, and the drive mechanism 12 for applying a driving force to the supporting member 11 so that the elongated medical instrument 1 moves toward the front-end side, the drive mechanism 12 including the elastic body 13; supporting the elongated medical instrument 1 with the supporting member 11 (see Fig. 1); and forcing the elongated medical instrument 1 to move toward the front-end side by stretching the elastic body 13 of the drive mechanism 12 (see Fig. 3) and then applying a restoring force of the stretched elastic body 13 to the supporting member (see Fig. 4).

As shown in Fig. 4, the flange 16a of the rod 16 included in the supporting member 11 collides with the rear end 2b of the catheter 2 when the elongated medical instrument 1 moves toward the front-end side. Therefore, the elongated medical instrument 1 will not move further toward the front-end side (from a position shown in Fig. 4).

In the elongated medical instrument-possessing medical device shown in Fig. 1, the flange 16a of the rod 16 included in the supporting member 11 is fixed to the elastic body 13. This allows the elastic body to subsequently contract to return the original state or shape (an unloaded state or shape in which no force is applied to the elastic body as shown in Fig. 1), and thus allows the elongated medical instrument 1 moved toward the front-end side as shown in Fig. 3 to return to the configuration as shown in Fig. 1.

Use of the elongated medical instrument-driving device 10, the elongated medical instrument-possessing medical device 100, and the method of driving an elongated medical instrument using the medical device 100 as shown in Fig. 1 enables a predetermined force (a large force equal to or higher than a certain value) or a predetermined velocity (a high velocity equal to or higher than a certain value) to be stably applied to the elongated medical instrument via the supporting member 11. The drive mechanism 12 included in the elongated medical instrument-driving device 10 has a simple configuration where the elastic body 13 is used. For this reason, the elongated medical instrument-driving device 10, the elongated medical instrument-possessing medical device 100, and the method of driving an elongated medical instrument using the medical device 100 may be advantageously used, for example, in a hospital not well equipped with facilities such as a power source for driving an elongated medical instrument.

A medical practitioner holds with her/his hand a portion of the rear-end side of the elongated medical instrument 1 and causes the front-end portion thereof to reach an occluded segment or place the front-end portion thereof at a position in the vicinity of the occluded segment. Subsequently, a medical catheter is delivered along the elongated medical instrument 1, where appropriate. After the rear end of the elongated medical instrument 1 is inserted into a through-hole disposed at the center of the supporting member 11 and the supporting member 11 is moved toward the front-end side, the supporting member 11 is used to support and fix the elongated medical instrument 1. The elastic body 13 of the drive mechanism 12 is stretched by holding and pulling the flange 16a of the rod 16 included in the supporting member 11 (see Fig. 3) backward with a hand. Subsequently, by releasing the hand from the flange 16a, a restoring force of the stretched elastic body 13 is applied to the supporting member 11 to move the elongated medical instrument 1 toward the front-end side along with the supporting member 11 (see Fig. 4). The front-end portion of the elongated medical instrument moved toward the front-end side will intrude into the occluded segment. By repeating these operations, the elongated medical instrument 1 can penetrate the occluded segment. Various therapeutic devices (for example, a balloon catheter) will be then delivered along the elongated medical instrument 1 to treat the occluded segment.

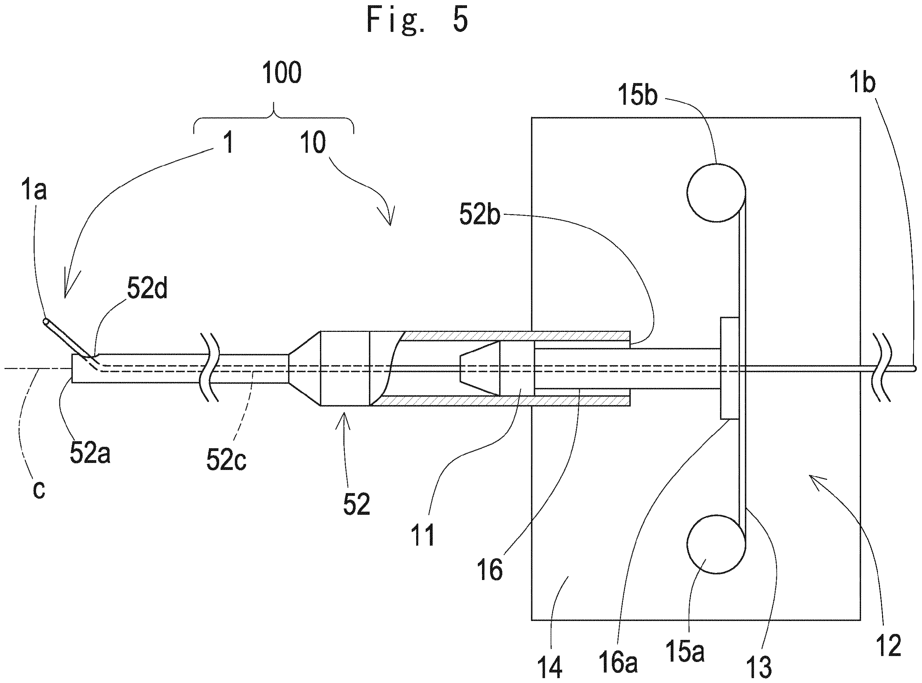

Fig. 5 shows a top view for illustrating an alternative method of using the elongated medical instrument-driving device 10 and the elongated medical instrument-possessing medical device 100 shown in Fig. 1.

The elongated medical instrument-driving device 10 and the elongated medical instrument-possessing medical device 100 can be used in a state where they are received in the inside of a medical catheter 52.

The medical catheter 52 has a front end 52a and a rear end 52b. The catheter 52 intrudes a through-hole 52c for receiving the elongated medical instrument 1 (for example, a medical guide wire). The front end of the through-hole 52c is connected to an opening 52d disposed on the outer peripheral surface of the catheter 52. Therefore, a portion of the front-end side of the elongated medical instrument 1 projects from the opening 52d and is arranged so as to incline against the axis direction (longitudinal direction) of the catheter. The axis direction (longitudinal direction) of the catheter is shown in Fig. 5 and indicated by the symbol c. Use of the catheter 52 enables the front end of the elongated medical instrument 1 to penetrate an inner membrane from a false lumen of a blood vessel to re-enter into a true lumen (the inner cavity of a blood vessel). Detailed description of the configuration of a medical catheter (including a through-hole having an opening on the outer peripheral surface) for use in such reentry, which is known, shall be omitted.

A medical practitioner delivers a medical guide wire, for example, as the elongated medical instrument 1 to an occluded segment, and forces a front end 1a thereof to intrude into a false lumen of a blood vessel and move forward (move toward the front-end side of the elongated medical instrument 1) until passing the occluded segment. Subsequently, the medical catheter 52 is delivered along the elongated medical instrument 1. A medical practitioner holds with her/his hand a portion of the rear-end side of the elongated medical instrument 1 and, such that a portion of the front end thereof is directed to the inner membrane of the blood vessel, rotates the elongated medical instrument 1 or the catheter 52 around the axis direction (longitudinal direction) thereof, if required. After the rear end of the elongated medical instrument 1 is inserted into a through-hole disposed at the center of the supporting member 11 and the supporting member 11 is moved toward the front-end side, the supporting member 11 is used to support and fix the elongated medical instrument 1. The elastic body 13 of the drive mechanism 12 is stretched by holding and pulling the flange 16a of the rod 16 included in the supporting member 11 backward with a hand. Subsequently, by releasing the hand from the flange, a restoring force of the stretched elastic body 13 is applied to the supporting member 11 to move the elongated medical instrument 1 toward the front-end side along with the supporting member 11. The front end of the elongated medical instrument moved toward the front-end side is moved toward the inner membrane, specifically toward a direction inclined against the axis direction c (longitudinal direction c) of the elongated medical instrument 1, and thus penetrates the inner membrane from the false lumen to re-enter (reentry) the true lumen (the inner cavity of the blood vessel). Various therapeutic devices (for example, a balloon catheter) will be then delivered along the elongated medical instrument 1 to treat the occluded segment.

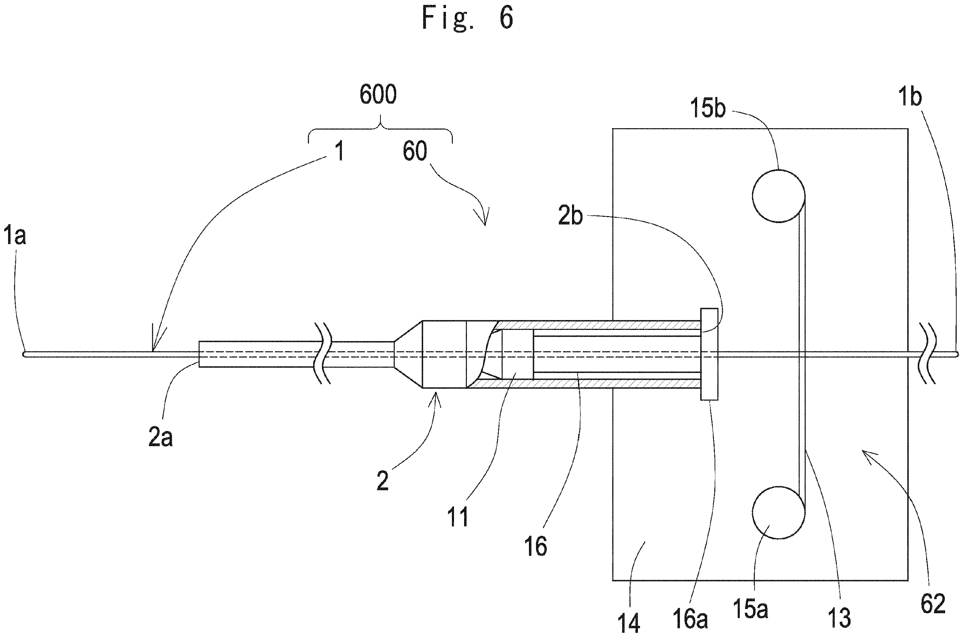

Fig. 6 shows a top view for illustrating a modified example (Modified Example A1) of the elongated medical instrument-driving device 10 and the elongated medical instrument-possessing medical device 100 shown in Fig. 1.

The configurations of an elongated medical instrument-driving device 60 and an elongated medical instrument-possessing medical device 600 are similar to those of the elongated medical instrument-driving device 10 and the elongated medical instrument-possessing medical device 100 shown in Figs. 1 to 4, respectively, except that the flange 16a of the rod 16 included in the supporting member 11 of a drive mechanism 62 is not fixed to the elastic body 13.

In the elongated medical instrument-driving device 60 of Fig. 6, the same components as those of the elongated medical instrument-driving device 10 of Fig. 1, which are already described, are given the same reference symbols without redundant descriptions.

In the elongated medical instrument-driving device 10 of Fig. 1, the flange 16a of the rod 16 included in the supporting member 11 is fixed to the elastic body 13. Therefore, the elastic body will subsequently contract to return the original state or shape (an unloaded state or shape in which no force is applied to the elastic body as shown in Fig. 1), and thus the elongated medical instrument 1 moved toward the front-end side as shown in Fig. 4 will return to the configuration as shown in Fig. 1.

In contrast, in the elongated medical instrument-driving device 60 of Fig. 6, the flange 16a of the rod 16 included in the supporting member 11 is not fixed to the elastic body 13. Therefore, the elastic body will subsequently contract to return the original state or shape (an unloaded state or shape in which no force is applied to the elastic body as shown in Fig. 6), and thus the elongated medical instrument 1 moved toward the front-end side as shown in Fig. 6 will maintain the configuration as shown in Fig. 6. That is, the elongated medical instrument 1 which is driven by the elongated medical instrument-driving device 60 to move toward the front-end side will remain in a resting state.

The elongated medical instrument-driving device 60 and the elongated medical instrument-possessing medical device 600 of Fig. 6 can exhibit similar effects as the elongated medical instrument-driving device 10 and the elongated medical instrument-possessing medical device 100 of Fig. 1, respectively.

The elongated medical instrument-driving device 60 of Fig. 6 is configured such that the supporting member 11 is detachable (separable) from the drive mechanism 62. Therefore, when the elongated medical instrument 1 has moved toward the front-end side, the supporting member 11 can be detached (separated) from the drive mechanism 62.

Use of the elongated medical instrument-driving device 60 can avoid application of an unnecessary (extremely large) driving force to an elongated medical instrument when, for example, the hardness of an occluded segment in a blood vessel is very large, and it is difficult to force the front end of a guide wire to intrude into the occluded segment (or force to move forward). That is, a driving force (extremely large driving force) to force an elongated medical instrument to move further toward the front-end side will not be applied to the elongated medical instrument whose movement toward the front-end side has become difficult.

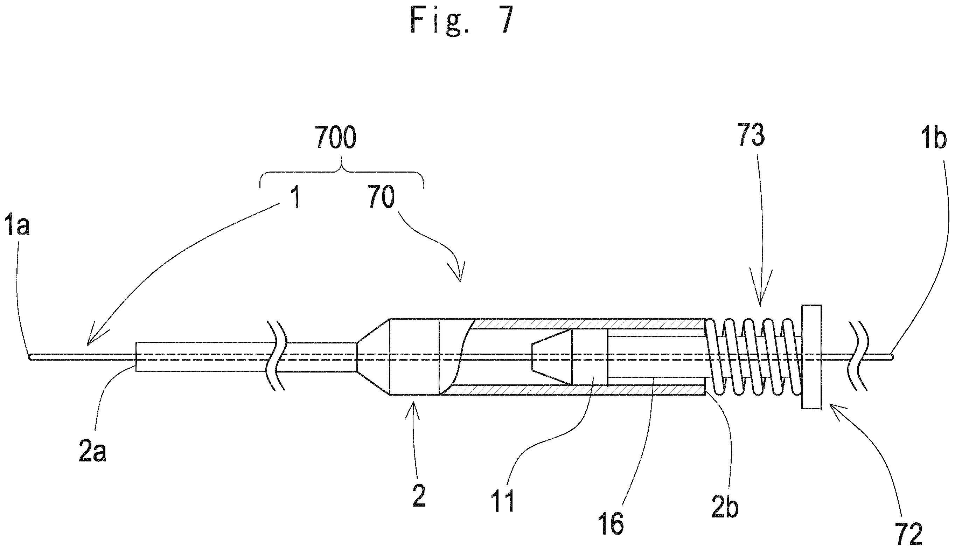

Fig. 7 shows a top view for illustrating another modified example (Modified Example A2) of the elongated medical instrument-driving device 10 and the elongated medical instrument-possessing medical device 100 shown in Fig. 1.

The configurations of an elongated medical instrument-driving device 70 and an elongated medical instrument-possessing medical device 700 are similar to those of the elongated medical instrument-driving device 10 and the elongated medical instrument-possessing medical device 100 shown in Figs. 1 to 4, respectively, except that a coil spring is used as an elastic body 73 inclined in a drive mechanism 72, and a pair of supporting pillars and a supporting plate which a driving device 70 is supported with (seated on) are not included.

In the elongated medical instrument-driving device 70 of Fig. 7, the same components as those of the elongated medical instrument-driving device 10 of Fig. 1, which have been already described, are given the same reference symbols without redundant descriptions.

A method of driving the elongated medical instrument 1 using the medical device 700 includes the steps of: providing the elongated medical instrument-possessing medical device 700 including the elongated medical instrument 1 having the front end 1a and the rear end 1b, the supporting member 11 for supporting the elongated medical instrument 1, and the drive mechanism 72 for applying a driving force to the supporting member 11 so that the elongated medical instrument 1 moves toward the front-end side, the drive mechanism 72 including the elastic body 73, the elastic body 73 including a coil spring; supporting the elongated medical instrument 1 with the supporting member 11; forcing the elongated medical instrument 1 to move toward the front-end side by stretching the elastic body 73 of the drive mechanism 72 and then applying a restoring force of the stretched elastic body 73 to the supporting member 11.

The elastic body 73 of the drive mechanism 72 can be stretched, for example, by holding the catheter 2 with the left hand, and holding the flange 16a of the rod 16 with the right hand to pull it backward. When the right hand is released from the flange 16a, a restoring force of the stretched elastic body 73 is applied to the supporting member 11, forcing the elongated medical instrument 1 to move forward (toward the front-end side of the elongated medical instrument 1) along with the supporting member 11.

The elongated medical instrument-driving device 70 and the elongated medical instrument-possessing medical device 700 of Fig. 7 can exhibit similar effects as the elongated medical instrument-driving device 10 and the elongated medical instrument-possessing medical device 100 of Fig. 1, respectively.

Use of a coiled spring as the elastic body 73 enables the elongated medical instrument to move more accurately toward the front-end side as compared with a case where a rubber string is used and also enables conferring of higher thermal resistance on the drive mechanism 72, since the elastic body 73 is resistant against thermal deterioration. No particular problem is anticipated even when the drive mechanism 72 is subjected to heat sterilization.

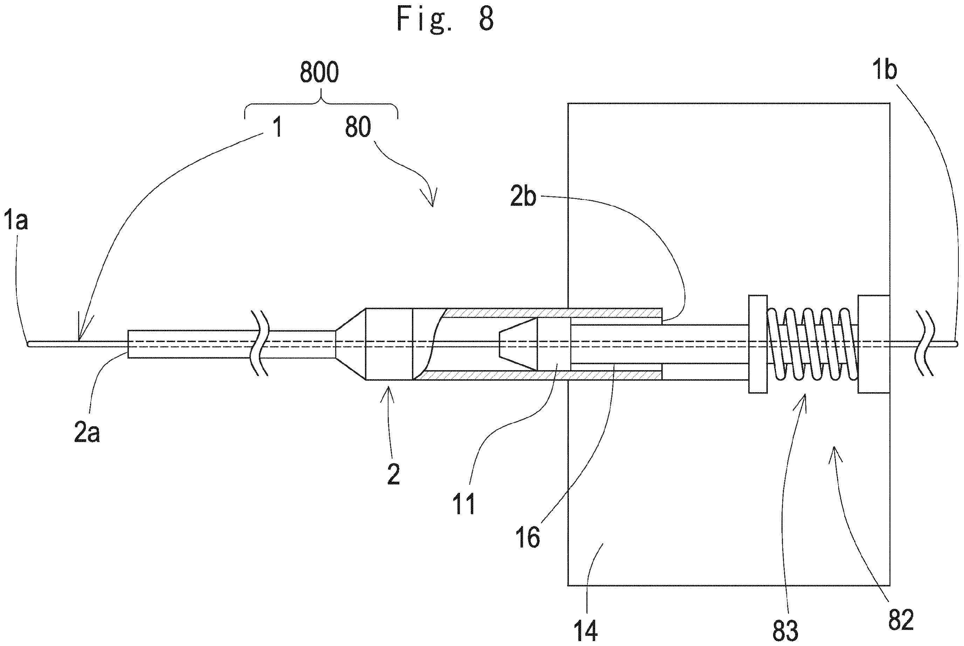

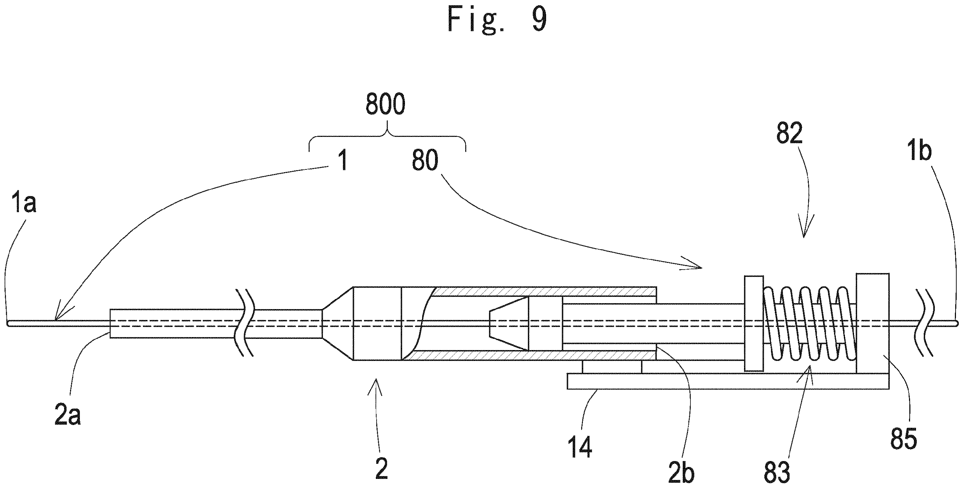

Fig. 8 shows a top view for illustrating yet another modified example (Modified Example A3) of the elongated medical instrument-driving device 10 and the elongated medical instrument-possessing medical device 100 shown in Fig. 1. Fig. 9 shows a side view of an elongated medical instrument-driving device 80 shown in Fig. 8.

The configurations of the elongated medical instrument-driving device 80 and an elongated medical instrument-possessing medical device 800 are similar to those of the elongated medical instrument-driving device 10 and the elongated medical instrument-possessing medical device 100 shown in Figs. 1 to 4, respectively, except that a coil spring is used as an elastic body 83 included in a drive mechanism 82, and the elastic body 83 is disposed between the flange 16a of the rod 16 and a supporting pillar 85 disposed on the surface of the supporting plate 14.

In the elongated medical instrument-driving device 80 of Fig. 8, the same components as those of the elongated medical instrument-driving device 10 of Fig. 1, which have been already described, are given the same reference symbols without redundant descriptions.

A method of driving the elongated medical instrument 1 using the medical device 800 includes the steps of: providing the elongated medical instrument-possessing medical device 800 including the elongated medical instrument 1 having the front end 1a and the rear end 1b, the supporting member 11 for supporting the elongated medical instrument 1, and the drive mechanism 82 for applying a driving force to the supporting member 11 so that the elongated medical instrument 1 moves toward the front-end side; supporting the elongated medical instrument with the supporting member 11; and forcing the elongated medical instrument 1 to move toward the front-end side by compressing the elastic body 83 of the drive mechanism 82 and then applying a restoring force of the compressed elastic body 83 to the supporting member 11.

As described above, the restoring force of the compressed elastic body 83 can also be applied to the supporting member 11.

As described above, the elastic body 83 of the drive mechanism 82 may be compressed, for example, by holding the supporting plate 14 with the left hand and holding the flange 16a of the rod 16 with the right hand to pull it backward. When the right hand is released from the flange 16a, a restoring force of the compressed elastic body 73 is applied to the supporting member 11, forcing the elongated medical instrument 1 (along with the supporting member 11) to move forward (toward the front-end side of the elongated medical instrument 1).

The driving device 80 and the elongated medical instrument-possessing medical device 800 can exhibit similar effects as the driving device 70 and the elongated medical instrument-possessing medical device 700 of Fig. 7.

In the driving device 80 of Fig. 8, the elastic body 83 is not stretched when a medical practitioner holds the flange 16c of the rod 16 and pulls it backward (moves it backward). This can prevent occurrence of plastic deformation of the elastic body 83 due to stretching.

### Embodiment B-1: Elongated medical instrument-driving device

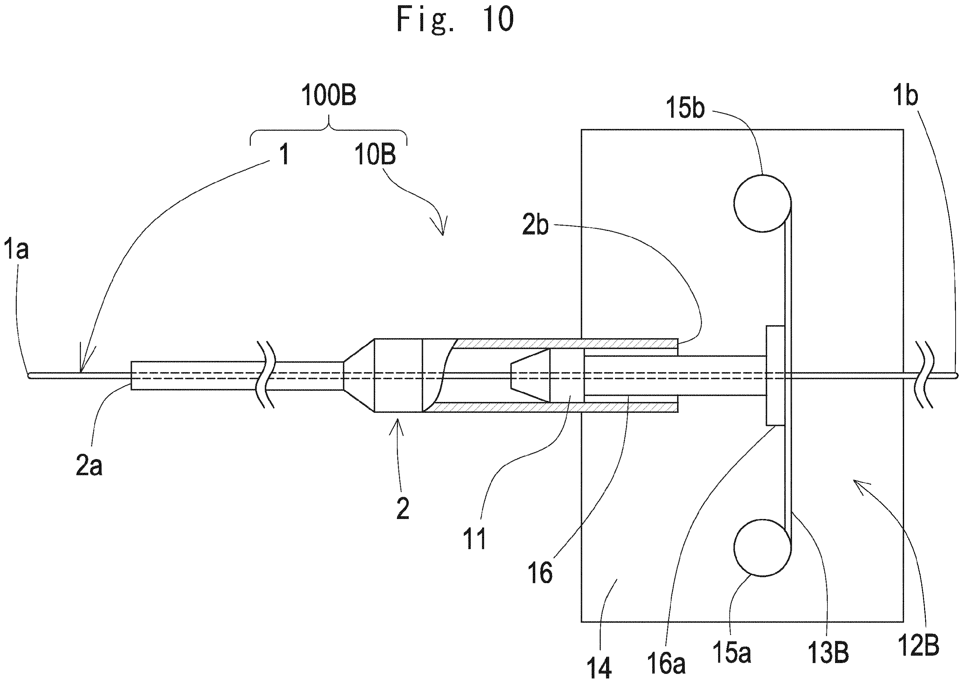

Fig. 10 shows a top view of the configuration of an elongated medical instrument-driving device (Embodiment B-1).

An elongated medical instrument-driving device 10B of Fig. 10 includes the supporting member 11 for supporting (or holding) the elongated medical instrument 1 having the front end 1a and the rear end 1b; and a drive mechanism 12B for applying a driving force to the supporting member 11 so that the elongated medical instrument 1 moves toward the front-end side.

The drive mechanism 12B is for applying an impact force or an impulse force to the supporting member 11.

A rubber string is used as an elastic body 13B of the drive mechanism 12B, and the rubber string is adjusted so that an impact force or an impulse force can be applied to the supporting member 11. Adjustment of the rubber string may be performed by appropriately adjusting, for example, the material of the rubber string or the shape or dimensions of the rubber string such as length, thickness, or width while checking if an impact force or an impulse force can be applied to the supporting member. Such adjustment can be performed without any particular problems nor undue experimentation.

The configurations of the elongated medical instrument-driving device 10B and the elongated medical instrument-possessing medical device 100B are similar to those of the elongated medical instrument-driving device 10 and the elongated medical instrument-possessing medical device 100 shown in Fig. 1, respectively, except that a rubber string is used as the elastic body 13B included in the drive mechanism 12B, the rubber string being adjusted so that an impact force or an impulse force can be applied to the supporting member 11.

In the elongated medical instrument-driving device 10B of Fig. 10, the same components as those of the elongated medical instrument-driving device 10 of Fig. 1, which are already described, are given the same reference symbols without redundant descriptions.

A known drive mechanism may be used as the drive mechanism 12B in place of a drive mechanism including an elastic body as described above as long as an impact force or an impulse force can be applied to the supporting member 11. Examples of the drive mechanism can include a linear-motion drive device (representative example: a linear motor) or a drive device in which a rotary drive device (representative example: a motor) is combined with a transducer for transforming a rotary motion of the rotary drive device into linear motion (representative example: a feed screw). The output of a linear-motion drive device or a rotary drive device is adjusted so that an impact force or an impulse force can be applied to the supporting member 11. Adjustment of the output of a linear-motion drive device or a rotary drive device may be performed, for example, by adjusting the electric power to be supplied to the linear-motion drive device or the rotary drive device while checking if an impact force or an impulse force can be applied to the supporting member. Adjustment of the electric power includes adjusting a current, voltage, or frequency to be supplied to a linear-motion drive device or a rotary drive device. Such adjustment can be performed without any particular problems nor undue experimentation.

### Embodiment B-2: Elongated medical instrument-possessing medical device

The elongated medical instrument-possessing medical device 100B includes the elongated medical instrument 1 and the elongated medical instrument-driving device 10B shown in Fig. 10.

The elongated medical instrument-possessing medical device 100B includes the elongated medical instrument 1 having the front end 1a and the rear end 1b, the supporting member 11 for supporting (or holding) the elongated medical instrument 1, and the drive mechanism 12B for applying a driving force to the supporting member 11 so that the elongated medical instrument 1 moves toward the front-end side, the drive mechanism 12B being for applying an impact force or an impulse force to the supporting member 11.

The configurations of the elongated medical instrument 1 and the elongated medical instrument-driving device 10B are as described above.

### Example B-3: Method of driving elongated medical instrument (not being part of the invention)

A method of driving an elongated medical instrument includes the steps of: providing the elongated medical instrument-possessing medical device 100B including the elongated medical instrument 1 having the front end 1a and the rear end 1b, the supporting member 11 for supporting (or holding) the elongated medical instrument 1, and the drive mechanism 12B for applying a driving force to the supporting member 11 so that the elongated medical instrument 1 moves toward the front-end side, the drive mechanism 12B being for applying an impact force or an impulse force to the supporting member 11; supporting the elongated medical instrument 1 with the supporting member 11 (see Fig. 10); and forcing the elongated medical instrument 1 to move toward the front-end side by applying an impact force or an impulse force to the supporting member 11 by the drive mechanism 12B.

Use of the elongated medical instrument-driving device 10B, the elongated medical instrument-possessing medical device 100B, and the method of driving an elongated medical instrument using the medical device 100B shown in Fig. 10 enables an impact force or an impulse force to be stably applied to the elongated medical instrument 1 via the supporting member 11.

A medical practitioner holds with her/his hand a portion of the rear-end side of the elongated medical instrument 1 and causes the front-end portion thereof to reach an occluded segment or place the front-end portion thereof at a position in the vicinity of the occluded segment. Subsequently, a medical catheter is delivered along the elongated medical instrument 1, where appropriate. After the rear end of the elongated medical instrument 1 is inserted into a through-hole disposed at the center of the supporting member 11 and the supporting member 11 is moved toward the front-end side, the supporting member 11 is used to support and fix the elongated medical instrument 1. The elastic body 13B of the drive mechanism 12B is stretched by holding the flange 16a of the rod 16 included in the supporting member 11 with a hand to pull it backward. Subsequently, an impact force or an impulse force is applied to the supporting member 11 when the stretched elastic body 13B contracts to return to the original state or shape (an unloaded state or shape in which no force is applied to the elastic body as shown in Fig. 10) by releasing the hand from the flange. When the elongated medical instrument 1 moves toward the front-end side along with the supporting member 11, and reaches an occluded segment, an impact force or an impulse force is applied to the occluded segment. This can allow the front end of the elongated medical instrument 1 to intrude more easily into the occluded segment. By repeating these operations, the elongated medical instrument 1 can penetrate the occluded segment. Various therapeutic devices (for example, a balloon catheter) will be then delivered along the elongated medical instrument 1 to treat the occluded segment.

Application of an impact force or an impulse force to the supporting member 11 enables a large force (energy) to be applied in a short time (while the supporting member 11 advances for a short distance) to the supporting member 11 or the elongated medical instrument 1 via the supporting member 11 without forcing these to move for a longer distance than necessary. That is, large movement is not required for accelerating the elongated medical instrument 1. By making the travel distance for acceleration smaller, it can reduce a risk in that the front-end portion of the elongated medical instrument 1 is brought into contact with a portion outside a treatment target on the wall of a blood vessel.

Once the front-end portion of the elongated medical instrument 1 moved toward the front-end side along with the supporting member 11 reaches an occluded segment (or once it is in a state where the front end has already reached and contacted with an occluded segment), a large energy applied to the supporting member is applied to the occluded segment as an impact force or an impulse force via the front end of the elongated medical instrument.

The elongated medical instrument-driving device 10B and the elongated medical instrument-possessing medical device 100B shown in Fig. 10 can also be used for forcing the front end of the elongated medical instrument 1 to penetrate an inner membrane from a false lumen of a blood vessel to re-enter (reentry) a true lumen (the inner cavity of a blood vessel). A method of achieving this is similar to that described with reference to Fig. 10, except that the elongated medical instrument-driving device 10B and the elongated medical instrument-possessing medical device 100B shown in Fig. 10 are used.

Similar to the elongated medical instrument-driving device 60 and the elongated medical instrument-possessing medical device 600 shown in Fig. 6, the elongated medical instrument-driving device 10B and the elongated medical instrument-possessing medical device 100B shown in Fig. 10 can exhibit similar effects by adopting a configuration where the flange 16a of the rod 16 included in the supporting member 11 of the drive mechanism 12B is not fixed to an elastic body 13C.

Similar to the elongated medical instrument-driving device 70 and the elongated medical instrument-possessing medical device 700 shown in Fig. 7 or the elongated medical instrument-driving device 80 and the elongated medical instrument-possessing medical device 800 shown in Fig. 8, the elongated medical instrument-driving device 10B and the elongated medical instrument-possessing medical device 100B shown in Fig. 10 can exhibit similar effects by adopting a configuration where a coil spring is used as the elastic body 13B included in the drive mechanism 12B.

### Embodiment C-1: Elongated medical instrument-driving device

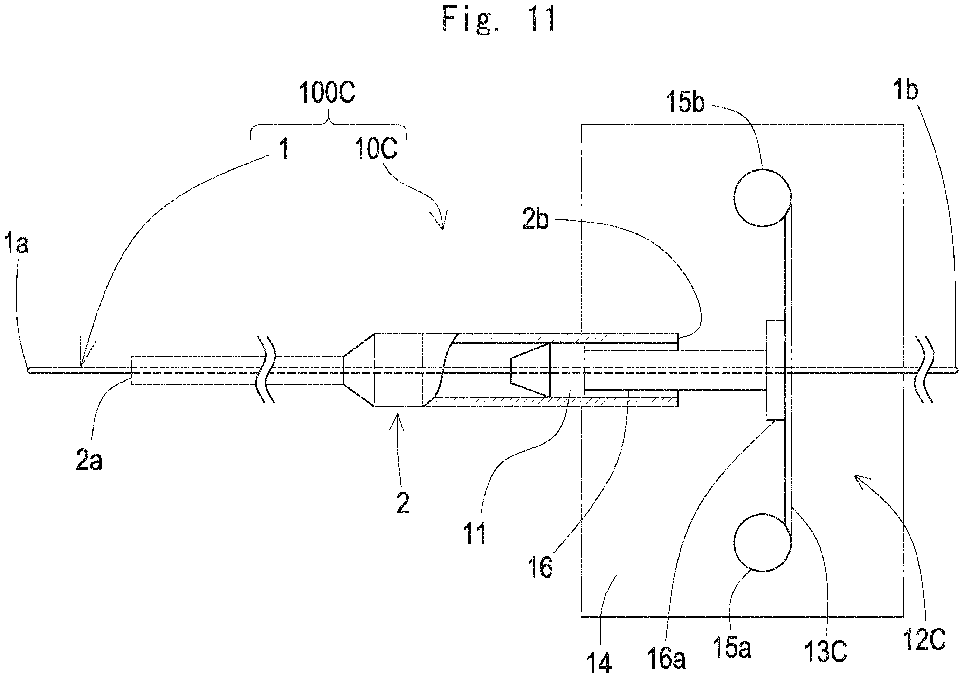

Fig. 11 shows a top view of the configuration of an elongated medical instrument-driving device (Embodiment C-1).

An elongated medical instrument-driving device 10C of Fig. 11 includes the supporting member 11 for supporting (or holding) the elongated medical instrument 1 having the front end 1a and the rear end 1b; and a drive mechanism 12C for applying a driving force to the supporting member 11 so that the elongated medical instrument 1 moves toward the front-end side.

The driving device 10C is such that the drive mechanism 12C can apply a driving force to the supporting member so that the velocity of the supporting member 11 falls within a range between 1 m/s and 30 m/s.

A rubber string is used as the elastic body 13C of the drive mechanism 12C. The rubber string is adjusted to be able to apply a driving force to the supporting member 11 so that the velocity of the supporting member 11 falls within a range between 1 m/s and 30 m/s. Adjustment of the rubber string is performed by appropriately adjusting, for example, the material of the rubber string or the shape or dimensions of the rubber string such as length, thickness, or width so that the velocity of the supporting member 11 falls within a range between 1 m/s and 30 m/s while checking the velocity of the supporting member. Such adjustment can be performed without any particular problems nor undue experimentation.

The velocity of the supporting member is set within a range between 1 m/s and 30 m/s, preferably within a range between 2 m/s and 25 m/s, more preferably within a range between 3 m/s and 23 m/s, and particularly preferably within a range between 4 m/s and 20 m/s.

The configurations of the elongated medical instrument-driving device 10C and an elongated medical instrument-possessing medical device 100C is similar to those of the elongated medical instrument-driving device 10 and the elongated medical instrument-possessing medical device 100 shown in Fig. 1, respectively, except that a rubber string is used as the elastic body 13C included in the drive mechanism 12C, the rubber string being adjusted to apply a driving force to the supporting member 11 so that the velocity of the supporting member 11 falls within a range between 1 m/s and 30 m/s.

In the elongated medical instrument-driving device 10C of Fig. 11, the same components as those of the elongated medical instrument-driving device 10 of Fig. 1, which have been already described, are given the same reference symbols without redundant descriptions.

A known drive mechanism may be used as the drive mechanism 12C in place of the aforementioned drive mechanism including the elastic body 13C as long as a driving force can be applied to the supporting member 11 so that the velocity of the supporting member 11 falls within a range between 1 m/s and 30 m/s (which hereinafter may also be referred to as the "driving force for achieving a velocity in a predetermined range").Examples of the drive mechanism can include a linear-motion drive device (representative example: a linear motor) or a drive device in which a rotary drive device (representative example: a motor) is combined with a transducer for transforming a rotary motion of the rotary drive device into linear motion (representative example: a feed screw). The output of a linear-motion drive device or a rotary drive device is adjusted so that the driving force for achieving a velocity in a predetermined range is applied to the supporting member 11. Adjustment of the output of a linear-motion drive device or a rotary drive device may be performed, for example, by adjusting the electric power to be supplied to the linear-motion drive device or the rotary drive device while checking if the driving force for achieving a velocity in a predetermined range can be applied to the supporting member. Adjustment of the electric power includes adjusting a current, voltage, or frequency to be supplied to a linear-motion drive device or a rotary drive device. Such adjustment can be performed without any particular problems nor undue experimentation.

The travel distance d of the supporting member may be set at a value satisfying the expression: 0 mm < d < 15 mm. The travel distance d of the supporting member may be set at a value satisfying the expression 0 mm < d < 10 mm, or may be set at a value satisfying the expression: 0 mm < d < 5 mm, or may be set at a value satisfying the expression: 0 mm < d < 3 mm, or may be set at a value satisfying the expression: 0 mm < d < 1 mm.

In view of achieving very short movement of the elongated medical instrument, the travel distance d of the supporting member may be set at a value satisfying the expression: 0.01 mm < d < 15 mm. The travel distance d of the supporting member may be set at a value satisfying the expression 0.01 mm < d < 10 mm, or may be set at a value satisfying the expression: 0.01 mm < d < 5 mm, or may be set at a value satisfying the expression: 0.01 mm < d < 3 mm, or may be set at a value satisfying the expression: 0.01 mm < d < 1 mm.

If the travel distance of the supporting member is too short, it may become difficult, for example, to force a guide wire used as the elongated medical instrument to intrude into an occluded segment. If the travel distance is too long, the time period during which a load (a force transmitted from the drive mechanism to a blood vessel via the supporting member, the guide wire, and the occluded segment) is applied to a blood vessel via the occluded segment may become long.

For example, the elongated medical instrument-driving device 10C of Fig. 11 has a configuration (which hereinafter may also be referred to as the "brake of the supporting member") where the supporting member cannot move further forward when the flange 16a of the rod 16 included in the supporting member 11 is brought into contact with the end portion of the catheter 2. There is no particular limitation for the configuration of the brake of the supporting member, and the brake of the supporting member may control the drive mechanism to limit the movement of the supporting member or may limit the movement of the supporting member by a stopper provided further on the front-end side with respect to the supporting member, with which the supporting member is to be brought into contact, collide, or engage.

### Embodiment C-2: Elongated medical instrument-possessing medical device

The elongated medical instrument-possessing medical device 100C includes the elongated medical instrument 1 and the elongated medical instrument-driving device 10C shown in Fig. 11.

The elongated medical instrument-possessing medical device 100C includes the elongated medical instrument 1 having the front end 1a and the rear end 1b, the supporting member 11 for supporting (or holding) the elongated medical instrument 1, and the drive mechanism 12C for applying a driving force to the supporting member 11 so that the elongated medical instrument 1 moves toward the front-end side, the drive mechanism 12C being for applying a driving force to the supporting member so that the velocity of the supporting member falls within a range between 1 m/s to 30 m/s.

The configurations of the elongated medical instrument 1 and the elongated medical instrument-driving device 10C are as described above.

### Example C-3: Method of driving elongated medical instrument (not being part of the invention)

A method of driving an elongated medical instrument includes the steps of: providing the elongated medical instrument-possessing medical device 100C including the elongated medical instrument 1 having the front end 1a and the rear end 1b, the supporting member 11 for supporting (or holding) the elongated medical instrument 1, and the drive mechanism 12C for applying a driving force to the supporting member 11 so that the elongated medical instrument 1 moves toward the front-end side, the drive mechanism being for applying a driving force to the supporting member 11 so that the velocity of the supporting member falls within a range between 1 m/s to 30 m/s; supporting the elongated medical instrument 1 with the supporting member 11 (see Fig. 11); and forcing the elongated medical instrument 1 to move toward the front-end side by applying the driving force of the drive mechanism 12C to the supporting member 11.

Use of the elongated medical instrument-driving device 10C, the elongated medical instrument-possessing medical device 100C, and the method of driving an elongated medical instrument using the medical device 100C shown in Fig. 11 enables a large force within a predetermined range or a high velocity within a predetermined range to be stably applied to the elongated medical instrument 1 via the supporting member 11.

A medical practitioner holds with her/his hand a portion of the rear-end side of the elongated medical instrument 1 and causes the front-end portion thereof to reach an occluded segment or place the front-end portion thereof at a position in the vicinity of the occluded segment. Subsequently, a medical catheter is delivered along the elongated medical instrument 1, where appropriate. After the rear end of the elongated medical instrument 1 is inserted into a through-hole disposed at the center of the supporting member 11 and the supporting member 11 is moved toward the front-end side, the supporting member 11 is used to support and fix the elongated medical instrument 1. The elastic body 13C of the drive mechanism 12C is stretched by holding the flange 16a of the rod 16 included in the supporting member 11 with a hand to pull it to the rear side. Subsequently, when the stretched elastic body 13C contracts to return to the original state or shape (an unloaded state or shape in which no force is applied to the elastic body as shown in Fig. 11) by releasing the hand from the flange, a driving force is applied to the supporting member 11 so that the velocity of the supporting member 11 falls within a range between 1 m/s and 30 m/s. The elongated medical instrument 1 moves toward the front-end side along with the supporting member 11 in a relatively high velocity (at a velocity within a range between 1 m/s and 30 m/s) to reach an occluded segment, and the front-end portion thereof then collides with the occluded segment. A relatively large force (energy) is applied to the occluded segment from the elongated medical instrument due to such collision. This can allow the front end of the elongated medical instrument 1 to intrude more easily into the occluded segment. By repeating these operations, the elongated medical instrument 1 can penetrate the occluded segment. Various therapeutic devices (for example, a balloon catheter) will be then delivered along the elongated medical instrument 1 to treat the occluded segment.

The elongated medical instrument-driving device 10C and the elongated medical instrument-possessing medical device 100C shown in Fig. 11 can also be used for forcing the front end of the elongated medical instrument 1 to penetrate an inner membrane from a false lumen of a blood vessel to re-enter (reentry) a true lumen (the inner cavity of a blood vessel). A method of achieving this is similar to that described with reference to Fig. 5, except that the elongated medical instrument-driving device 10C and the elongated medical instrument-possessing medical device 100C shown in Fig. 11 are used.

Similar to the elongated medical instrument-driving device 60 and the elongated medical instrument-possessing medical device 600 shown in Fig. 6, the elongated medical instrument-driving device 10C and the elongated medical instrument-possessing medical device 100C shown in Fig. 11 can exhibit similar effects by adopting a configuration where the flange 16a of the rod 16 included in the supporting member 11 of the drive mechanism 12C is not fixed to the elastic body 13C.

Similar to the elongated medical instrument-driving device 70 and the elongated medical instrument-possessing medical device 700 shown in Fig. 7 or the elongated medical instrument-driving device 80 and the elongated medical instrument-possessing medical device 800 shown in Fig. 8, the elongated medical instrument-driving device 10C and the elongated medical instrument-possessing medical device 100C shown in Fig. 11 can exhibit similar effects by adopting a configuration where a coil spring is used as the elastic body 13C included in the drive mechanism 12B.

### Embodiment D-1: Elongated medical instrument-driving device

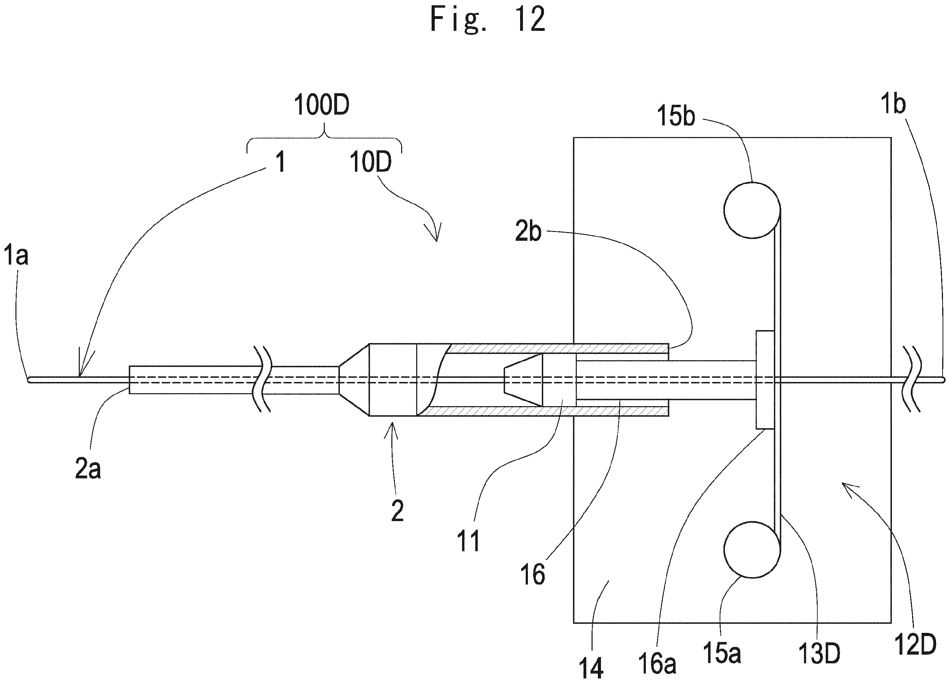

Fig. 12 shows a top view of the configuration of an elongated medical instrument-driving device (Embodiment D-1).

An elongated medical instrument-driving device 10D of Fig. 12 includes the supporting member 11 for supporting (or holding) the elongated medical instrument 1 having the front end 1a and the rear end 1b; and a drive mechanism 12D for applying a driving force to the supporting member 11 so that the elongated medical instrument 1 moves toward the front-end side.

The drive mechanism 12D is for applying a driving force (which hereinafter may also be referred to as a "driving force for enabling inertial movement of the supporting member") large enough to enable the supporting member 11 to move (move inertially) further toward the front-end side to the supporting member 11.

A rubber string is used as an elastic body 13D of the drive mechanism 12D. The rubber string is adjusted so that a driving force for enabling inertial movement of the supporting member 11 is applied to the supporting member 11.

Adjustment of the rubber string is performed by appropriately adjusting, for example, the material of the rubber string or the shape or dimensions of the rubber string such as length, thickness, or width while checking if the supporting member 11 moves (moves inertially) further toward the front end when application of the driving force to the supporting member 11 is stopped. Such adjustment can be performed without any particular problems nor undue experimentation.

In the case of the driving device 10D, application of the driving force to the supporting member 11 is stopped once the elastic body 13D is stretched as in the case of the driving device 10 shown in Fig. 3, and the stretched elastic body 13D is then allowed to contract to return to the original state or shape (the same configuration as an unloaded state or shape in which no force is applied to the elastic body for the elastic body 13 shown in Fig. 1). Subsequently, in the case of the driving device 10D, the supporting member 11 moves (moves inertially) further toward the front-end side as in the case of the driving device 10 shown in Fig. 4.

The configurations of the elongated medical instrument-driving device 10D and a elongated medical instrument-possessing medical device 100D are similar to those of the elongated medical instrument-driving device 10 and the elongated medical instrument-possessing medical device 100 shown in Fig. 1, respectively, except that a rubber string is used as the elastic body 13D included in the drive mechanism 12D, the rubber string being adjusted so that the driving force for enabling inertial movement of the supporting members 11 as described above is applied to the supporting member 11.

In the elongated medical instrument-driving device 10D of Fig. 12, the same components as those of the elongated medical instrument-driving device 10 of Fig. 1, which have been already described, are given the same reference symbols without redundant descriptions.

A known drive mechanism may be used as the drive mechanism 12D in place of the aforementioned drive mechanism including the elastic body 13D as long as it enables inertial movement of the member 11 as described above. Examples of the drive mechanism can include a linear-motion drive device (representative example: a linear motor) or a drive device in which a rotary drive device (representative example: a motor) is combined with a transducer for transforming a rotary motion of the rotary drive device into linear motion (representative example: a feed screw). The output of a linear-motion drive device or a rotary drive device is adjusted so as to enable inertia movement of the supporting member 11. Adjustment of the output of a linear-motion drive device or a rotary drive device may be performed, for example, by adjusting the electric power to be supplied to the linear-motion drive device or the rotary drive device while checking if the supporting member can be moved inertially as described above. Adjustment of the electric power includes adjusting a current, voltage, or frequency to be supplied to a linear-motion drive device or a rotary drive device. Such adjustment can be performed without any particular problems nor undue experimentation.

### Embodiment D-2: Elongated medical instrument-possessing medical device

The elongated medical instrument-possessing medical device 100D includes the elongated medical instrument 1 and the elongated medical instrument-driving device 10D shown in Fig. 12.

The elongated medical instrument-possessing medical device 100D includes the elongated medical instrument 1 having the front end 1a and the rear end 1b, the supporting member 11 for supporting (or holding) the elongated medical instrument 1, and the drive mechanism 12D for applying a driving force to the supporting member 11 so that the elongated medical instrument 1 moves toward the front-end side, the drive mechanism 12D being for applying a driving force to the supporting member 11, the driving force being large enough to enable the supporting member 11 to move further toward the front-end side when application of the driving force to the supporting member 11 is stopped.

The configurations of the elongated medical instrument 1 and the elongated medical instrument-driving device 10D are as described above.

### Example D-3: Method of driving elongated medical instrument (not being part of the invention)

A method of driving an elongated medical instrument includes the steps of: providing the elongated medical instrument-possessing medical device 100D including the elongated medical instrument 1 having the front end 1a and the rear end 1b, the supporting member 11 for supporting (or holding) the elongated medical instrument 1, and the drive mechanism 12D for applying a driving force to the supporting member 11 so that the elongated medical instrument 1 moves toward the front-end side, the drive mechanism 12D being for applying a driving force to the supporting member 11, the driving force being large enough to enable the supporting member 11 to move further toward the front-end side when application of the driving force to the supporting member 11 is stopped; supporting the elongated medical instrument 1 with the supporting member (see Fig. 12); and forcing the elongated medical instrument 1 to move toward the front-end side by applying the driving force of the drive mechanism 12D to the supporting member 11.

In the elongated medical instrument-driving device 10D, the elongated medical instrument-possessing medical device 100D, and the method of driving an elongated medical instrument using the medical device 100D shown in Fig. 12, the supporting member 11 can move further toward the front-end side when application of the driving force to the supporting member 11 is stopped, but during this, no driving force is applied to the supporting member 11 from the drive mechanism 12D.

Use of the elongated medical instrument-driving device 10D can, for example, in a case where the degree of hardness of an occluded segment in a blood vessel is extremely high such that it is difficult for the front end of a guide wire to intrude into the occluded segment (difficult to move forward), application of an unnecessary driving force (a driving force more than necessary to move an elongated medical instrument toward the front-end side) to an elongated medical instrument moving toward the front-end side can be avoided when application of driving force to the supporting member 11 from the drive mechanism 12D is stopped. That is, a driving force (extremely large driving force) to force an elongated medical instrument to move further toward the front-end side will not be applied to an elongated medical instrument whose movement toward the front-end side has become difficult.

A medical practitioner holds with her/his hand a portion of the rear-end side of the elongated medical instrument 1 and causes the front-end portion thereof to reach an occluded segment or place the front-end portion thereof at a position in the vicinity of the occluded segment. Subsequently, a medical catheter 2 is delivered along the elongated medical instrument 1, where appropriate. After the rear end of the elongated medical instrument 1 is inserted into a through-hole disposed at the center of the supporting member 11 and the supporting member 11 is moved toward the front-end side, the supporting member 11 is used to support and fix the elongated medical instrument 1. The elastic body 13D of the drive mechanism 12D is stretched by holding the flange 16a of the rod 16 included in the supporting member 11 with a hand to pull it backward. Subsequently, when the stretched elastic body 13D contacts to return to the original state or shape (an unloaded state or shape in which no force is applied to the elastic body as shown in Fig. 12) by releasing the hand from the flange 16a, application of a driving force to the supporting member 11 is stopped, but the supporting member 11 moves (moves inertially) further toward the front-end side. The elongated medical instrument 1 inertially moves toward the front-end side along with the supporting member 11 in a relatively high velocity to reach an occluded segment, and the front-end portion thereof then collides with the occluded segment. A relatively large force (energy), such as a large force (energy) which enables inertial movement of the elongated medical instrument 1 and the supporting member 11, is applied to the occluded segment from the elongated medical instrument due to such collision. For example, a large force (energy) is applied for enabling inertial movement of the elongated medical instrument 1 and the supporting member 11. This can allow the front end of the elongated medical instrument 1 to intrude more easily into the occluded segment. By repeating these operations, the elongated medical instrument 1 can penetrate the occluded segment. Various therapeutic devices (for example, a balloon catheter) will be then delivered along the elongated medical instrument 1 to treat the occluded segment.

The elongated medical instrument-driving device 10D and the elongated medical instrument-possessing medical device 100D shown in Fig. 12 can also be used for forcing the front end of the elongated medical instrument 1 to penetrate an inner membrane from a false lumen of a blood vessel to re-enter (reentry) a true lumen (the inner cavity of a blood vessel). A method of achieving this is similar to that described with reference to Fig. 5, except that the elongated medical instrument-driving device 10D and the elongated medical instrument-possessing medical device 100D shown in Fig. 12 are used.

Similar to the elongated medical instrument-driving device 60 and the elongated medical instrument-possessing medical device 600 shown in Fig. 6, the elongated medical instrument-driving device 10D and the elongated medical instrument-possessing medical device 100D shown in Fig. 12 can exhibit similar effects by adopting a configuration where the flange 16a of the rod 16 included in the supporting member 11 of the drive mechanism 12D is not fixed to the elastic body 13D.

Similar to the elongated medical instrument-driving device 70 and the elongated medical instrument-possessing medical device 700 shown in Fig. 7 or the elongated medical instrument-driving device 80 and the elongated medical instrument-possessing medical device 800 shown in Fig. 8, the elongated medical instrument-driving device 10D and the elongated medical instrument-possessing medical device 100D shown in Fig. 12 can exhibit similar effects by adopting a configuration where a coil spring is used as the elastic body 13D included in the drive mechanism 12D.

### REFERENCE SIGNS LIST

- 1: elongated medical instrument
1a front end (of elongated medical instrument)
1b rear end (of elongated medical instrument)
- 2: catheter
2a front end
2b rear end
- 10: driving device
- 11: supporting member
- 12: driving mechanism
- 13: elastic body
- 14: supporting plate
- 15a: supporting pillar
- 15b: supporting pillar
- 16: rod
16a flange
- 52: catheter
52a front end
52b rear end
52c through-hole
52d opening
- 100: medical device

## Claims

1. An elongated medical instrument-driving device (10) comprising:
a supporting member (11) for supporting an elongated medical instrument (1) having a front end (1a) and a rear end (1b); and
a drive mechanism (12) for applying a driving force to the supporting member (11) so that the elongated medical instrument (1) moves toward a front-end side together with the supporting member (11),
wherein the drive mechanism (12) is configured to apply the predetermined force or the predetermined velocity to the elongated medical instrument (1).

2. An elongated medical instrument-driving device (10) according to claim 1, wherein the drive mechanism (12) includes an elastic body (13) applying the drive force to the supporting member (11).

3. An elongated medical instrument-driving device (10) according to claim 2, wherein the elastic body (13) includes a rubber string, a coil spring, or a flat spring

4. An elongated medical instrument-driving device (10) according to claim 1, wherein the drive mechanism (12) is configured to apply an impact force or an impulse force to the supporting member (11).

5. An elongated medical instrument-driving device (10) according to claim 1, wherein the drive mechanism is configured to apply a driving force to the supporting member (11) so that the velocity of the supporting member (11) falls within a range between 1 m/s and 30 m/s.

6. An elongated medical instrument-driving device (10) according to claim 1, wherein,
the drive mechanism (12) is configured to apply a driving force to the supporting member (11) so that the supporting member (11) moves toward a front-end side, and
the driving force is large enough to enable the supporting member (11) to move further toward a front-end side when application of the driving force to the supporting member (11) is stopped.

7. An elongated medical instrument-driving device (10) according to any one of claims 1 to 6, wherein the supporting member (11) is configured to be movable by being separated or detached from the drive mechanism (12) when the driving force is applied.

8. An elongated medical instrument-driving device (10) according to any one of claims 1 to 7, wherein the elongated medical instrument (1) is a medical guide wire or catheter.

9. A medical device (100) comprising:
an elongated medical instrument (1) having a front end and a rear end; and
an elongated medical instrument-driving device (10) according to any one of claims 1 to 8.

## Patentansprüche

1. Antriebsvorrichtung (10) für ein langgestrecktes medizinisches Instrument, mit:
einem Tragelement (11) zum Tragen eines langgestreckten medizinischen Instruments (1) mit einem vorderen Ende (1a) und einem hinteren Ende (1b) und
einem Antriebsmechanismus (12) zum Aufbringen einer Antriebskraft auf das Tragelement (11), so dass sich das langgestreckte medizinische Instrument (1) zusammen mit dem Tragelement (11) zu einer Vorderendseite hin bewegt,
wobei der Antriebsmechanismus (12) so eingerichtet ist, dass er die vorbestimmte Kraft oder die vorbestimmte Geschwindigkeit auf das langgestreckte medizinische Instrument (1) aufbringt.

2. Antriebsvorrichtung (10) für ein langgestrecktes medizinisches Instrument nach Anspruch 1, bei der der Antriebsmechanismus (12) einen elastischen Körper (13) aufweist, der die Antriebskraft auf das Tragelement (11) aufbringt.

3. Antriebsvorrichtung (10) für ein langgestrecktes medizinisches Instrument nach Anspruch 2, bei der der elastische Körper (13) eine Gummischnur, eine Spiralfeder oder eine Flachfeder aufweist.

4. Antriebsvorrichtung (10) für ein langgestrecktes medizinisches Instrument nach Anspruch 1, bei der der Antriebsmechanismus (12) so eingerichtet ist, dass er eine Stoßkraft oder eine Impulskraft auf das Tragelement (11) aufbringt.

5. Antriebsvorrichtung (10) für ein langgestrecktes medizinisches Instrument nach Anspruch 1, bei der der Antriebsmechanismus so eingerichtet ist, dass er eine Antriebskraft auf das Tragelement (11) aufbringt, so dass die Geschwindigkeit des Tragelements (11) in einen Bereich zwischen 1 m/s und 30 m/s fällt.

6. Antriebsvorrichtung (10) für ein langgestrecktes medizinisches Instrument nach Anspruch 1, bei der
der Antriebsmechanismus (12) so eingerichtet ist, dass er eine Antriebskraft auf das Tragelement (11) aufbringt, so dass sich das Tragelement (11) zu einer Vorderendseite hin bewegt, und
die Antriebskraft ausreichend groß ist, damit sich das Tragelement (11) weiter zu einer Vorderendseite hin bewegen kann, wenn die Aufbringung der Antriebskraft auf das Tragelement (11) angehalten wird.

7. Antriebsvorrichtung (10) für ein langgestrecktes medizinisches Instrument nach einem der Ansprüche 1 bis 6, bei der das Tragelement (11) so eingerichtet ist, dass es bewegbar ist, indem es vom Antriebsmechanismus (12) getrennt oder abgenommen wird, wenn die Antriebskraft aufgebracht wird.

8. Antriebsvorrichtung (10) für ein langgestrecktes medizinisches Instrument nach einem der Ansprüche 1 bis 7, bei der das langgestreckte medizinische Instrument (1) ein medizinischer Führungsdraht oder Katheter ist.

9. Medizinische Vorrichtung (100) mit:
einem langgestreckten medizinischen Instrument (1) mit einem vorderen Ende und einem hinteren Ende und
einer Antriebsvorrichtung (10) für ein langgestrecktes medizinisches Instrument nach einem der Ansprüche 1 bis 8.

## Revendications

1. Dispositif d'entraînement (10) d'un instrument médical allongé, comprenant :
un élément support (11) pour supporter un instrument médical allongé (1) présentant une extrémité avant (1a) et une extrémité arrière (1b) ; et
un mécanisme d'entraînement (12) pour exercer une force d'entraînement sur l'élément support (11) de sorte que l'instrument médical allongé (1) se déplace ensemble avec l'élément support (11) en direction d'un côté d'extrémité avant,
le mécanisme d'entraînement (12) étant réalisé de manière à exercer la force prédéterminée ou la vitesse prédéterminée sur l'instrument médical allongé (1).

2. Dispositif d'entraînement (10) d'un instrument médical allongé selon la revendication 1, dans lequel le mécanisme d'entraînement (12) présente un corps élastique (13) exerçant la force d'entraînement sur l'élément support (11).

3. Dispositif d'entraînement (10) d'un instrument médical allongé selon la revendication 2, dans lequel le corps élastique (13) présente un cordon en caoutchouc, un ressort hélicoïdal ou un ressort plat.

4. Dispositif d'entraînement (10) d'un instrument médical allongé selon la revendication 1, dans lequel le mécanisme d'entraînement (12) est réalisé de manière à exercer une force d'impact ou une force d'impulsion sur l'élément support (11).

5. Dispositif d'entraînement (10) d'un instrument médical allongé selon la revendication 1, dans lequel le mécanisme d'entraînement est réalisé de manière à exercer une force d'entraînement sur l'élément support (11), de sorte que la vitesse de l'élément support (11) se situe dans une plage comprise entre 1 m/s et 30 m/s.

6. Dispositif d'entraînement (10) d'un instrument médical allongé selon la revendication 1, dans lequel
le mécanisme d'entraînement (12) est réalisé de manière à exercer une force d'entraînement sur l'élément support (11) de sorte que l'élément support (11) se déplace en direction d'un côté d'extrémité avant, et
la force d'entraînement est suffisamment élevée pour permettre le déplacement de l'élément support (11) plus loin en direction d'un côté d'extrémité avant lorsque l'exercice de la force d'entraînement sur l'élément support (11) est stoppé.

7. Dispositif d'entraînement (10) d'un instrument médical allongé selon l'une des revendications 1 à 6, dans lequel l'élément support (11) est réalisé de manière à pouvoir être déplacé en étant séparé ou détaché du mécanisme d'entraînement (12) lorsque la force d'entraînement est exercée.

8. Dispositif d'entraînement (10) d'un instrument médical allongé selon l'une des revendications 1 à 7, dans lequel l'instrument médical allongé (1) est un fil de guidage médical ou un cathéter.

9. Dispositif médical (100), comprenant :
un instrument médical allongé (1) présentant une extrémité avant et une extrémité arrière ; et
un dispositif d'entraînement (10) d'un instrument médical allongé selon l'une des revendications 1 à 8.
